# EUROPEAN PATENT APPLICATION

(11) **EP 3 967 298 A1**
(43) Date of publication of application: **16.03.2022**
(21) Application number: 21182872.8
(22) Date of filing: 30.06.2021
(51) Int. Cl.: A61K 9/08, A61K 9/12, A61K 31/439, A61K 47/10, A61P 25/34, A24B 15/167

(54) **AEROSOL COMPOSITION FOR ORAL USE**

(30) Priority: 30.06.2020 PL 43451920
(71) Applicant: Uniwersytet Rzeszowski, 35-959 Rzeszów (PL); Podkarpackie Centrum Innowacji sp. z o.o., 35-051 Rzeszów (PL)
(72) Inventor: Tutka, Piotr, 20-554 Lublin (PL); Czernicka-Kubicka, Anna, 35-601 Rzeszów (PL); Wróblewski, Karol, 37-114 Bia obrzegi (PL); Ko odziejczyk, Patrycjusz, 35-106 Rzeszów (PL); Bieniasz, Mateusz, 37-205 Zarzecze (PL)
(74) Representative: Bartula-Toch, Marta

(57) **Abstract**

An oral aerosol composition comprising: cytisine in an amount of 0.01 to 10 wt%, a hydrophilic non-ionic emulsifier from the group of copolymers or polymers in an amount of 0.1 to 10 wt%, at least one alcohol in an amount of 0.01 to 40 wt%, excipients and purified water making a total of 100 wt%, wherein the composition has a pH of 7 to 10.

## Description

The invention relates to an oral aerosol composition for use in the treatment of nicotine addiction.

The most common form of treatment for nicotine addiction is nicotine replacement therapy, which involves replacing the nicotine in tobacco with nicotine in another form. However, it is not accepted by some smokers, it has limited effectiveness and it can aggravate addiction. Varenicline and bupropion therapies are also used, which in turn are relatively expensive and, as prescription drugs, they require medical appointments, which makes them not readily available for many smokers. Moreover, varenicline and bupropion therapies are associated with various side effects.

Previous studies have shown that cytisine may provide an effective, safe and cost-efficient alternative to other nicotine replacement therapies. Currently, cytisine is available in the form of oral tablets or capsules. However, this way of dosing is inconvenient for patients since it's necessary to take the medication very often, which significantly reduces the likeliness that this form of therapy is opted for, and reduces patient compliance, which in turn makes the therapy less effective.

Studies show poor compliance in more than half of patients treated with cytisine tablets. In addition, this form is not appropriate for many patients suffering from gastrointestinal diseases.

WO2016/060576A1 publication discloses a liquid for an electronic cigarette containing cytisine in an amount of 0.000000001% to 99.999999999% by volume of the solution. Also, EP2815741A1 publication discloses the use of cytisine as a nicotine substitute in liquids. CN101428021A publication discloses the external use of cytisine in the form of creams, gels or sprays aimed at reducing nicotine craving. PT2361081T publication discloses a preparation containing cytisine administered orally in the form of chewing gum.

The essence of the solution of the invention is that the aerosol composition comprises the following: cytisine in an amount of 0.01 to 10 wt%, a hydrophilic non-ionic emulsifier from the group of copolymers or polymers in an amount of 0.1 to 10 wt%, at least one alcohol in an amount of 0.01 to 40 wt%, excipients and purified water making a total of 100 wt%, wherein the composition has a pH of 7 to 10. The invention is characterised in that it comprises pH-adjusting agents (other than cytisine) that contribute to an appropriate environment for drug absorption.

Preferably, the composition comprises cytisine in the form of a pure compound or a salt thereof.

Preferably, the hydrophilic non-ionic emulsifier is selected from the group consisting of: Poloxamer 407, polysorbates and PEG 400.

Preferably, the alcohols belong to the group comprising: ethanol (99.8%), propylene glycol, polyethylene glycol, glycerol.

Preferably, the excipients are flavour and aroma enhancers in an amount of 0.1 to 5 wt% and pH-adjusting agents.

In a preferred embodiment, the flavour enhancer is menthol.

Preferably, the aroma enhancer is mint flavouring.

Preferably, the aroma enhancer is a fruit flavouring.

In a preferred embodiment, the flavour enhancer is sucralose.

The flavour enhancer is preferably a mixture of erythritol and steviol glycosides in a ratio of 99.5 : 0.5.

Preferably, the pH-adjusting agents belong to the group consisting of hydrochloric acid, sodium bicarbonate and tris(hydroxymethyl)aminomethane hydrochloride.

The results of scientific research demonstrate a significant advantage in the efficacy of aerosols over conventional forms of drugs, such as tablets. The aerosol accelerates the absorption of cytisine and thus relieves nicotine withdrawal symptoms, including nicotine craving. Reducing/alleviating nicotine craving prevents relapse and significantly increases abstinence rates.

The use of aerosol improves the safety profile and provides an improved tolerability of the substance compared to the hitherto used tablets. It is assumed that as a result of reduced risk of gastrointestinal complaints, the most common side effect of cytisine tablets, approximately 10-15% more smokers will be able to be treated with the drug in aerosol form. The administration of cytisine in aerosol form improves patient compliance, which results in an improvement and shortening of the treatment time, making it more cost-effective. Producing cytisine in an aerosol form increases efficacy, safety, and improves patient comfort and gives the patient the option to choose which form of the drug is more convenient for them. Moreover, the use of cytisine in aerosol form reduces the cost of treatment by approximately 20% compared to preparations containing nicotine, thus increasing the availability of the drug for smokers regardless of their social situation.

Another advantage of the invention over pills/capsules may be a quicker onset of alleviation/abatement of nicotine craving symptoms that are associated with cigarette withdrawal. Here, the onset of activity is crucial important since the most common reason for relapsing is nicotine craving and other nicotine withdrawal symptoms. Reducing this time prevents patients from reaching for a cigarette and returning to smoking. Additionally, administration of the drug in the aerosol form minimises the bitter taste in the mouth and throat. This was achieved by preparing various variants of formulations comprising flavour and aroma enhancers.

Selection of individual components of the composition and their amounts allows to obtain the desired physical and chemical properties of the preparation, including homogeneous content of active substance in the entire volume of the formulation, and, consequently, in each single dose of the drug. The carrier used in the formulation was selected so as to provide measurable benefits in the form of optimising its absorption.

The pH level of the preparation has been selected to ensure stability of the composition without causing irritation of the mucosa.

Hydrophilic non-ionic surfactants, not susceptible to changes in pH of the environment, were used for easier dissolution of the preparation ingredients that are poorly water-soluble.

Very importantly, no changes were found in the stability of the preparation or its organoleptically assessed characteristics when stored at room temperature.

The solution according to the invention is illustrated in the embodiments.

### Example I

| **Substance name** | **Composition** | | | | | |
|---|---|---|---|---|---|---|
| | **(a) [% wt.]** | **(b) [% wt.]** | **(c) [% wt.]** | **(d) [% wt.]** | **(e) [% wt.]** | **(f) [% wt.]** |
| Cytisine | 0.35 | 0.52 | 1.04 | 2.07 | 3.11 | 4.14 |
| Purified water | 54.96 | 54.79 | 54.27 | 53.24 | 52.20 | 51.17 |
| Poloxamer 407 | 4.00 | 4.00 | 4.00 | 4.00 | 4.00 | 4.00 |
| Propylene glycol | 15.00 | 15.00 | 15.00 | 15.00 | 15.00 | 15.00 |
| Glycerin | 2.50 | 2.50 | 2.50 | 2.50 | 2.50 | 2.50 |
| Mint flavouring or other flavours, e.g. fruity flavours | 0.03 | 0.03 | 0.03 | 0.03 | 0.03 | 0.03 |
| Menthol | 1.00 | 1.00 | 1.00 | 1.00 | 1.00 | 1.00 |
| Sucralose | 0.28 | 0.28 | 0.28 | 0.28 | 0.28 | 0.28 |
| Tris(hydroxyme thyl)aminometh ane hydrochloride | 4.05 | 4.05 | 4.05 | 4.05 | 4.05 | 4.05 |
| Sodium bicarbonate | 1.43 | 1.43 | 1.43 | 1.43 | 1.43 | 1.43 |
| Ethanol (99.8%) | 10.00 | 10.00 | 10.00 | 10.00 | 10.00 | 10.00 |
| Hydrochloric acid (1M) | 6.40 | 6.40 | 6.40 | 6.40 | 6.40 | 6.40 |

Poloxamer 407 was dissolved in purified water, followed by gradually adding the other ingredients under continuous mixing of the solution using a magnetic mixer. The pH of the solution was adjusted to 9.0 with hydrochloric acid (1M). The entire preparation procedure was carried out using solvents at room temperature without heating.

### Example 2

| **Substance name** | **Composition** | | | | | |
|---|---|---|---|---|---|---|
| | **(a) [% wt.]** | **(b) [% wt.]** | **(c) [% wt.]** | **(d) [% wt.]** | **(e) [% wt.]** | **(f) [% wt.]** |
| Cytisine | 0.35 | 0.52 | 1.04 | 2.07 | 3.11 | 4.14 |
| Purified water | 66.84 | 66.67 | 66.15 | 65.12 | 64.08 | 63.05 |
| Poloxamer 407 | 4.00 | 4.00 | 4.00 | 4.00 | 4.00 | 4.00 |
| Propylene glycol | 15.00 | 15.00 | 15.00 | 15.00 | 15.00 | 15.00 |
| Glycerin | 2.50 | 2.50 | 2.50 | 2.50 | 2.50 | 2.50 |
| Mint flavouring or other flavours, e.g. fruity flavours | 0.03 | 0.03 | 0.03 | 0.03 | 0.03 | 0.03 |
| Menthol | 1.00 | 1.00 | 1.00 | 1.00 | 1.00 | 1.00 |
| Sucralose | 0.28 | 0.28 | 0.28 | 0.28 | 0.28 | 0.28 |
| Ethanol (99.8%) | 10 | 10 | 10 | 10 | 10 | 10 |

Poloxamer 407 was dissolved in purified water, followed by gradually adding the other ingredients under continuous mixing of the solution using a magnetic mixer. The pH was adjusted to approximately 9.0. The entire preparation procedure was carried out using solvents at room temperature without heating.

### Example 3

| **Substance name** | **Com** | | | **position** | | |
|---|---|---|---|---|---|---|
| | **(a) [% wt.]** | **(b) [% wt.]** | **(c) [% wt.]** | **(d) [% wt.]** | **(e) [% wt.]** | **(f) [% wt.]** |
| Cytisine | 0.35 | 0.52 | 1.04 | 2.07 | 3.11 | 4.14 |
| Formulation water | 51.24 | 51.07 | 50.55 | 49.52 | 48.48 | 47.45 |
| Poloxamer 407 | 4.00 | 4.00 | 4.00 | 4.00 | 4.00 | 4.00 |
| Propylene glycol | 15.00 | 15.00 | 15.00 | 15.00 | 15.00 | 15.00 |
| Glycerin | 2.50 | 2.50 | 2.50 | 2.50 | 2.50 | 2.50 |
| Mint flavouring or other flavours, e.g. fruity flavours | 0.03 | 0.03 | 0.03 | 0.03 | 0.03 | 0.03 |
| Menthol | 1.00 | 1.00 | 1.00 | 1.00 | 1.00 | 1.00 |
| Erythritol | 3.98 | 3.98 | 3.98 | 3.98 | 3.98 | 3.98 |
| Steviol glycosides | 0.02 | 0.02 | 0.02 | 0.02 | 0.02 | 0.02 |
| Tris(hydroxymethyl) aminomethane hydrochloride | 4.05 | 4.05 | 4.05 | 4.05 | 4.05 | 4.05 |
| Sodium bicarbonate | 1.43 | 1.43 | 1.43 | 1.43 | 1.43 | 1.43 |
| Ethanol (99.8%) | 10 | 10 | 10 | 10 | 10 | 10 |
| Hydrochloric acid (1M) | 6.4 | 6.4 | 6.4 | 6.4 | 6.4 | 6.4 |

Poloxamer 407 was dissolved in purified water, followed by gradually adding the other ingredients under continuous mixing of the solution using a magnetic mixer. The pH of the solution was adjusted to 9.0 with hydrochloric acid (1M). The entire preparation procedure was carried out using solvents at room temperature without heating.

### Example 4

| **Substance name** | **Com** | | | **position** | | |
|---|---|---|---|---|---|---|
| | **(a) [% wt.]** | **(b) [% wt.]** | **(c) [% wt.]** | **(d) [% wt.]** | **(e) [% wt.]** | **(f) [% wt.]** |
| Cytisine | 0.35 | 0.52 | 1.04 | 2.07 | 3.11 | 4.14 |
| Poloxamer 407 | 4.00 | 4.00 | 4.00 | 4.00 | 4.00 | 4.00 |
| Propylene glycol | 15.00 | 15.00 | 15.00 | 15.00 | 15.00 | 15.00 |
| Glycerin | 2.50 | 2.50 | 2.50 | 2.50 | 2.50 | 2.50 |
| Mint flavouring or other flavours, e.g. fruity flavours | 0.03 | 0.03 | 0.03 | 0.03 | 0.03 | 0.03 |
| Menthol | 1.00 | 1.00 | 1.00 | 1.00 | 1.00 | 1.00 |
| Sucralose | 0.28 | 0.28 | 0.28 | 0.28 | 0.28 | 0.28 |
| Tris(hydroxymeth yl)aminomethane hydrochloride | 2.03 | 2.03 | 2.03 | 2.03 | 2.03 | 2.03 |
| Sodium bicarbonate | 0.72 | 0.72 | 0.72 | 0.72 | 0.72 | 0.72 |
| Ethanol (99.8%) | 7.90 | 7.90 | 7.90 | 7.90 | 7.90 | 7.90 |
| Propyl 4-hydroxybenzoate | 0.03 | 0.03 | 0.03 | 0.03 | 0.03 | 0.03 |
| Methyl 4-hydroxybenzoate | 0.07 | 0.07 | 0.07 | 0.07 | 0.07 | 0.07 |
| Hydrochloric acid | ad pH 9.0 | ad pH 9.0 | ad pH 9.0 | ad pH 9.0 | ad pH 9.0 | ad pH 9.0 |
| Purified water | q.s. | q.s. | q.s. | q.s. | q.s. | q.s. |

Poloxamer 407 was dissolved in purified water, followed by gradually adding the other ingredients (except for half the amount of ethanol) under continuous mixing of the solution using a magnetic mixer. The pH of the composition was lowered to 7.0 using hydrochloric acid solution, followed by adding parabens (previously dissolved in the remaining amount of ethanol). Purified water was added to achieve the required weight of the preparation. Propyl 4-hydroxybenzoate and methyl 4-hydroxybenzoate were used. Preferably, they are used in a ratio (1:2) with a concentration of at least 0.05%. In the example presented, a concentration of 0.1 wt% of these preservatives in the formulation was used.

### Example 5

| **Substance name** | **Composition** | | | | | |
|---|---|---|---|---|---|---|
| | **(a) [% wt.]** | **(b) [% wt.]** | **(c) [% wt.]** | **(d) [% wt.]** | **(e) [% wt.]** | **(f) [% wt.]** |
| Cytisine | 0.35 | 0.52 | 1.04 | 2.07 | 3.11 | 4.14 |
| Purified water | 54.95 | 54.78 | 54.26 | 53.23 | 52.19 | 51.16 |
| Poloxamer 407 | 4.00 | 4.00 | 4.00 | 4.00 | 4.00 | 4.00 |
| Propylene glycol | 15.00 | 15.00 | 15.00 | 15.00 | 15.00 | 15.00 |
| Glycerin | 2.50 | 2.50 | 2.50 | 2.50 | 2.50 | 2.50 |
| Mint flavouring or other flavours, e.g. fruity flavours | 0.03 | 0.03 | 0.03 | 0.03 | 0.03 | 0.03 |
| Menthol | 1.00 | 1.00 | 1.00 | 1.00 | 1.00 | 1.00 |
| Sucralose | 0.28 | 0.28 | 0.28 | 0.28 | 0.28 | 0.28 |
| Tris(hydroxyme thyl)aminometh ane hydrochloride | 4.05 | 4.05 | 4.05 | 4.05 | 4.05 | 4.05 |
| Sodium bicarbonate | 1.43 | 1.43 | 1.43 | 1.43 | 1.43 | 1.43 |
| 2,6-di-tert-butyl-4-methylphenol | 0.01 | 0.01 | 0.01 | 0.01 | 0.01 | 0.01 |
| Ethanol (99.8%) | 10.00 | 10.00 | 10.00 | 10.00 | 10.00 | 10.00 |
| Hydrochloric acid (1M) | 6.40 | 6.40 | 6.40 | 6.40 | 6.40 | 6.40 |

Poloxamer 407 was dissolved in purified water, followed by gradually adding the other ingredients under continuous mixing of the solution using a magnetic mixer. 2,6-di-tert-butyl-4-methylphenol must be dissolved in half the amount of ethanol provided in the composition before being added to the solution. The pH of the solution was adjusted to 9.0 with hydrochloric acid (1M). The entire preparation procedure was carried out using solvents at room temperature.

### Example 6

| **Substance name** | **Composition** | | | | | |
|---|---|---|---|---|---|---|
| | **(a) [% wt.]** | **(b) [% wt.]** | **(c) [% wt.]** | **(d) [% wt.]** | **(e) [% wt.]** | **(f) [% wt.]** |
| Cytisine succinate | 0.57 | 0.84 | 1.68 | 3.36 | 5.04 | 6.71 |
| Purified water | 54.74 | 54.47 | 53.63 | 51.95 | 50.27 | 48.60 |
| Poloxamer 407 | 4.00 | 4.00 | 4.00 | 4.00 | 4.00 | 4.00 |
| Propylene glycol | 15.00 | 15.00 | 15.00 | 15.00 | 15.00 | 15.00 |
| Glycerin | 2.50 | 2.50 | 2.50 | 2.50 | 2.50 | 2.50 |
| Mint flavouring or other flavours, e.g. fruity flavours | 0.03 | 0.03 | 0.03 | 0.03 | 0.03 | 0.03 |
| Menthol | 1.00 | 1.00 | 1.00 | 1.00 | 1.00 | 1.00 |
| Sucralose | 0.28 | 0.28 | 0.28 | 0.28 | 0.28 | 0.28 |
| Tris(hydroxyme thyl)aminometh ane hydrochloride | 4.05 | 4.05 | 4.05 | 4.05 | 4.05 | 4.05 |
| Sodium bicarbonate | 1.43 | 1.43 | 1.43 | 1.43 | 1.43 | 1.43 |
| Ethanol (99.8%) | 10.00 | 10.00 | 10.00 | 10.00 | 10.00 | 10.00 |
| Hydrochloric acid (1M) | 6.40 | 6.40 | 6.40 | 6.40 | 6.40 | 6.40 |

Poloxamer 407 was dissolved in purified water, followed by gradually adding the other ingredients under continuous mixing of the solution using a magnetic mixer. The pH of the solution was adjusted to 9.0 with hydrochloric acid (1M). The entire preparation procedure was carried out using solvents at room temperature without heating.

Solutions were prepared with varying amounts of cytisine in solution. One dose of the preparation was assumed to be 0.147 ml of solution. Composition (a) relates to a concentration of cytisine of 3.5 mg/g per dose of the preparation, composition (b) to 5.2 mg/g per dose of the preparation, composition (c) to 10.4 mg/g per dose of the preparation, composition (d) to 20.07 mg/g per dose of the preparation, composition (e) to 31.1 mg/g per dose of the preparation, and composition (f) to 41.4 mg/g per dose of the preparation.

The primary solvent used is water, with a secondary solvent being propylene glycol, which also serves as a solubilising agent and preservative. Moreover, propylene glycol serves as an aroma and flavour carrier. The glycerol used, in turn, serves as a cosurfactant, increases viscosity and provides a sweet taste. Additionally, ethanol is used as a second cosurfactant. It allows e.g. to dissolve ingredients that are water-insoluble (e.g. menthol or parabens) and then to add them to the solution. Sweeteners and menthol are added as flavour enhancers. Organoleptic tests were also useful in their selection.

Cytisine, as a weak organic base with pKa =7.92 in an acidic environment, is found mainly in an ionised form, which hinders absorption through biological membranes. At pH below 7.92, the dissociated form is prevalent. The pH of human saliva is usually in the range of 6.2 - 7.6 (6.7 on average), so that preferably a pH > 7 is achieved in order to shift the dissociation balance toward the neutral form, which is better absorbed through biological membranes. At a pH 2 units higher than pKa, an alkaline drug will be present in 99% in aqueous solutions in a non-ionised form. The use of pH with lower difference in relation to pKa allows to achieve sufficient drug absorption from oral mucosa. A suitable buffer medium (TRIS-HCl) was used to ensure a stable pH within the desired range. In accordance with the organoleptic tests conducted, the preparation does not irritate the oral cavity within the pH range of 7 to 10.

Cytisine solution is stored in orange glass (type III), HDPE or PET bottles equipped with a dosing pump with a PP or HDPE cap. The volume filled was checked by weight. The bottle was closed with a dosing pump (equipped with a valve with a spray head and nozzle) having a specific output volume containing the appropriate dose of cytisine. In the examples presented, a single aerosol dose contains 0.5 to 6 mg of cytisine, corresponding to concentrations in the range of 3.5 to 41.4 mg/g (compositions designated a to f). The preparations are preferably stored at room temperature.

## Claims

1. An oral aerosol composition **characterised in that** it comprises: cytisine in an amount of 0.01 to 10 wt%, a hydrophilic non-ionic emulsifier from the group of copolymers or polymers in an amount of 0.1 to 10 wt%, at least one alcohol in an amount of 0.01 to 40 wt%, excipients and purified water making a total of 100 wt%, wherein the composition has a pH of 7 to 10.

2. The composition according to claim 1 **characterised in that** it comprises cytisine in the form of a pure compound or a salt thereof.

3. The composition according to claim 1 **characterised in that** the hydrophilic non-ionic emulsifier is selected from the group comprising: Poloxamer 407, polysorbates and PEG 400.

4. The composition according to claim 1 or 2, **characterised in that** the alcohols belong to the group comprising: ethanol, propylene glycol, glycerol.

5. The composition according to claim 1 or 2 or 3, **characterised in that** the excipients are flavour and aroma enhancers in an amount 0.01 to 5 wt% and pH-adjusting agents.

6. The composition according to claim 7 **characterised in that** the flavour enhancer is menthol.

7. The composition according to claim 7 **characterised in that** the aroma enhancer is mint flavouring.

8. The composition according to claim 7 **characterised in that**, the aroma enhancer is a fruit flavouring.

9. The composition according to claim 7 **characterised in that** the flavour enhancer is sucralose.

10. The composition according to claim 7 **characterised in that** the flavour enhancer is a mixture of erythritol and steviol glycosides in a ratio of 99.5 : 0.5.

11. The composition according to claim 7 **characterised in that** the pH-adjusting agents are derived from the group consisting of hydrochloric acid, sodium bicarbonate and tris(hydroxymethyl)aminomethane hydrochloride.
